# EUROPEAN PATENT SPECIFICATION

(11) **EP 3 415 493 B1**
(45) Date of publication and mention of the grant of the patent: **01.01.2020**
(21) Application number: 17305744.9
(22) Date of filing: 16.06.2017
(51) Int. Cl.: C07C 45/48, C07C 49/76

(54) **PROCESS FOR THE CATALYTIC DECARBOXYLATIVE CROSS-KETONISATION OF ARYL- AND ALIPHATIC CARBOXYLIC ACIDS**
VERFAHREN ZUR KATALYTISCHEN DECARBOXYLIERENDE KREUZKETONISIERUNG VON ARYL- UND ALIPHATISCHEN CARBONSÄUREN
PROCÉDÉ DE CÉTONISATION CROISÉE CATALYTIQUE DÉCARBOXYLANTE D'ACIDES CARBOXYLIQUES ALIPHATIQUES ET ARYL

(43) Date of publication of application: 19.12.2018
(73) Proprietor: RHODIA OPERATIONS, 75009 Paris (FR)
(72) Inventor: BACK, Olivier, 69008 Lyon (FR); MARION, Philippe, 69390 Vernaison (FR)
(74) Representative: Delenne, Marc

(56) References cited:
- EP-A1- 2 468 708
- GB-A- 1 063 268
- US-A- 3 329 723
- "Decarboxylation studies. II Preparation of alkylphenyl Ketones", J. ORG. CHEM., vol. 28, 1963, pages 879-881, XP002775519,

## Description

### FIELD OF THE INVENTION

The present invention relates to a process for the preparation of aryl aliphatic ketones through catalytic decarboxylative cross-ketonisation of aryl carboxylic acids and aliphatic carboxylic acids (Piria reaction).

### BACKGROUND OF THE INVENTION

Processes for the decarboxylative cross-ketonisation of aryl carboxylic acids and alkyl carboxylic acids are known in the art. One may refer to the following prior art documents :
- US patent n° 2,697,729 published in 1954 describes at Example XII the reaction between stearic acid and benzoic acid in a nearly equimolar stoichiometry catalyzed by alumina Al₂O₃ in a closed vessel during 4h30 at 350°C. A poor selectivity is obtained. Indeed, only 30 % of the product is stearophenone, that is the aryl alkyl ketone, and 45 % of the product is stearone, that is the dialkyl ketone.
- US patent n°3,329,723 published in 1962 reports the synthesis of cyclohexyl-phenyl ketone through decarboxylative cross-ketonisation between cyclohexanecarboxylic acid and benzoic acid. The cross-ketonisation reaction is mediated by Mn^{(II)} salts. A catalytic molten media is first prepared by reacting at 122°C at least 1 equivalent of MnO with 2 equivalents of carboxylic acids. The reaction temperature is then increased at 345°C triggering ketonization. A mixture of carboxylic acids (cyclohexanecarboxylic acid: benzoic acid = 1.22:1) is continuously added into the reaction medium under a stream of either hydrogen or steam to keep the catalyst active.

Overall the amount of Mn metal used is around 6 mol % relative to the total amount of carboxylic acids engaged in the reaction.

A very good selectivity of 98 % of cyclohexyl-phenyl ketone has been obtained. However, the conversion regarding benzoic acid is 81 % and the conversion regarding cyclohexanecarboxylic acid is only 65 % according to example 1. Partial conversions of starting acids require tedious work-up that can be costly for industrialization. Furthermore, the necessity to conduct reaction under a stream of hydrogen or steam in combination with high temperature and acidic media is also detrimental for an industrial process. Finally it is specified in the text that "operating according to the same method, but replacing the manganese (II) by iron (II) or cadmium, also the mixed ketone is obtained, but the yields are not quantitative" showing that Mn salts are necessary and iron salts do not give good results. Using toxic Mn salts can be detrimental for industrialization of the process.
- The paper entitled *"*Decarboxylation studies. II Preparation of alkyl phenyl Ketones" published in J. Org. Chem., 28, pp.879-881, (1963) discloses a cross-ketonization process in which benzoic acid is reacted with an alkyl carboxylic acid in liquid phase in the presence of iron. During the reaction iron salts are first formed from Fe⁽⁰⁾ at 250°C during 45 min and the complexes are decomposed at 280-300°C during 3 h to form the aryl alkyl ketone. It is worth noting that slight excess of iron is used relative to the total molar quantities of benzoic and aliphatic acids. According to the experimental procedure described in the article, 0.1 mole of benzoic acid, 0.1 mole of aliphatic acid and 0.11 mole of Fe (corresponding to a 10 % mol excess) are used. The reaction thus requires a significant amount of iron. It is therefore desirable to find a process which uses lower amount of metal. Furthermore, Table I of this paper shows that the isolated yields of the heavier aryl alkyl ketones (containing between 9 and 17 carbon atoms in their alkyl chain) varies between 50 % and 66 %. The highest yield is 66 % (corresponding to an analyzed crude yield of 78 %) which is obtained by reacting benzoic acid with palmitic acid. This paper reports on Table II that using a 4-fold excess of benzoic acid with respect to propanoic acid leads to an isolated yield of 72 % of propiophenone. However, in the perspective of an industrial process, using a high excess of a reactant requires additional processing steps to remove and recycle the reactant in excess and therefore add additional costs. The yield drops to only 60 % when a 2-fold excess of benzoic acid is used. It is therefore desirable to find a process which allows preparing the aryl alkyl ketone at a higher yield, typically at least 80 % and with reduced amounts of metals and limited excess of reactant
- More recently, a catalytic decarboxylative cross-ketonisation of aryl- and alkyl-carboxylic acids has been described in Adv. Synth. Catal., 353, pp.57-63, (2011) and in EP-A- 2 468 708. Linear aliphatic carboxylic acids are reacted with 3-toluic acid in presence of a magnetite (Fe₃O₄) nanopowder. In this study, magnetite is used in catalytic amounts. With this catalyst, the cross-ketonization between m-toluic acid and phenylacetic acid affords a mixture of the desired cross-adduct and 1,3-diphenylacetone (the homo-ketonization product of phenylacetic acid) in a 10:1 ratio with a yield of 80 %. Table 3 shows that the reaction may lead to a yield of the aryl ketone between 74 and 86 % using a slight excess of aryl carboxylic acid with respect to the carboxylic acid (1.2:1). Nevertheless, one major drawback of this process is that it involves the use of a nanopowder which can be costly and difficult to handle in case of an implementation at an industrial scale. Besides, the reaction requires using a high boiling point solvent (Dowtherm A) which has then to be removed in a downstream process through distillation. This additional step of solvent removal results in an uneconomical process when run at an industrial scale. Besides, it is to be noted that the reaction time is 21h, which is very long.

In conclusion, in the above prior art documents, either the metal involved in the decarboxylative cross-ketonisation is employed in a stoichiometric amount (Fe) or is toxic and sensitive requiring complex implementation (e.g. in the case of Mn^{(II)} salts, the use of a stream of hydrogen or H₂O is necessary) or the process requires the use of expensive nanoparticles in combination with a high boiling point solvent. The use of a solvent can be detrimental for industrialization as separation of products will require complex and expensive downstream processes, namely distillation of high boiling point compounds. Also, the yields hardly exceed 85 % in those studies, and for the Mn mediated ketonization process the reactants conversion is partial requiring costly purification steps. Finally, the product mixture always contains a significant amount of homo-ketonization dialkyl ketones, which will require again additional purification steps.

Therefore, a process is sought which obviates the above-mentioned drawbacks.

### SUMMARY OF THE INVENTION

It has now been found a new process for the preparation of aryl aliphatic ketones through catalytic decarboxylative cross-ketonisation of aryl carboxylic acids and aliphatic carboxylic acids. This process uses a metal in a catalytic amount and allows preparing aryl aliphatic ketones with a high yield, typically at least 70 % and up to 99 %. A high selectivity in aryl aliphatic ketone may also be obtained, typically at least than 80 % and up to 99 %.

In a first object of the invention is a process for the catalytic decarboxylative cross-ketonisation of aryl- and aliphatic carboxylic acids comprising the steps of:
a) providing a mixture containing :
   i) an aryl carboxylic acid;
   ii) an aliphatic carboxylic acid;
   iii) a metal-containing compound;
   in which the number of moles of the metal in the mixture is at least equal to 90 % of the sum of the number of moles of aryl carboxylic acid and the number of moles of aliphatic carboxylic acid divided by the valency of the metal;
   said mixture being substantially free of any added solvent;
b) heating the mixture at a temperature sufficient to form metal carboxylates;
c) further heating the mixture at a temperature sufficient to form a dialiphatic ketone and an aryl aliphatic ketone;
d) adding to the reaction mixture of step c) :
   i) aryl carboxylic acid in an amount which corresponds substantially to the amount of aryl carboxylic acid consumed during the formation of the aryl aliphatic ketone in step c);
   ii) aliphatic carboxylic acid in an amount which corresponds substantially to the amount of aliphatic carboxylic acid consumed during the formation of the aryl aliphatic ketone and the dialiphatic ketone in step c);
   maintaining the mixture at a temperature sufficient to continue forming the dialiphatic ketone and the aryl aliphatic ketone;
e) optionally repeating step d).

According to one embodiment, the mixture substantially free of any added solvent is free of any added solvent (i.e. it is completely free of any added solvent).

According to one embodiment :
x is the number of moles of aryl carboxylic acid,
y is the number of moles of aliphatic carboxylic acid, and
the number of moles of a metal of valence z in the mixture of step a) is within the range of from 0.9[(x+y)/z] to 1.1[(x+y)/z], preferably from 0.95[(x+y)/z] to 1.05[(x+y)/z], more preferably from 0.98[(x+y)/z] to 1.02[(x+y)/z], more preferably from 0.99[(x+y)/z] to 1.01[(x+y)/z].

According to one embodiment, the metal is iron.

According to one embodiment, the iron-containing compound is selected from the group consisting of iron metal, bivalent Fe^{(II)} compounds, trivalent Fe^{(III)} compounds, compounds in which iron is present in both bivalent Fe^{(II)} and bivalent Fe^{(III)} oxidation states, such as magnetite Fe₃O₄.

According to one embodiment, step b) is carried out at a temperature T₁ from 225°C to 290°C, preferably from 250°C to 275°C.

According to one embodiment, step c) and/or step d) is carried out at a temperature T₂ from 300°C to 400°C, preferably from 315°C to 400°C.

According to one embodiment, the molar ratio of the total amount of aryl carboxylic acid and the aliphatic carboxylic acid ranges from 0.3 to 1.8.

According to one embodiment, heating in step b) is carried out for a duration ranging from 1 to 6 h, preferably for a duration less than or equal to 3h.

According to one embodiment, heating in step c) is carried out for a duration ranging from 1 to 4 h, preferably for a duration less than or equal to 1,5h.

According to one embodiment, heating in step d) is carried out for a duration ranging from 1 to 4 h, preferably for a duration less than or equal to 1,25 h.

According to one embodiment, the process is carried out for a duration less than or equal to 20 hours, preferably for a duration less than or equal to 8 hours.

According to one embodiment, the aryl carboxylic acid is selected from the group consisting of benzoic acid, furoic acid, o-toluic acid, m-toluic acid and p-toluic acid.

According to one embodiment, the aliphatic carboxylic acid is a fatty acid. The fatty acid may contain from 8 to 18 carbon atoms.

Another object of the invention is a process for the preparation of at least one end compound from at least one aryl aliphatic ketone, said method comprising :
- synthesizing the aryl aliphatic ketone through the process as described above,
- causing the aryl aliphatic ketone to react in accordance with a single or multiple chemical reaction scheme involving at least one reagent other than the aryl aliphatic ketone, wherein at least one product of the chemical reaction scheme is the end compound that is not further caused to be chemically converted into another compound.

### DETAILED DESCRIPTION OF THE PREFERRED EMBODIMENTS

In the following description, the term "yield in ketone" refers to the ratio between the total number of moles of aryl aliphatic ketone plus twice of the total number of moles of dialiphatic ketones produced through the process of the invention over the overall number of moles of aliphatic carboxylic acid.

The term "selectivity in aryl aliphatic ketone" refers to the ratio between the number of moles of aryl aliphatic ketone produced through the process of the invention and the number of moles of all the ketones produced (mainly aryl aliphatic ketone and dialiphatic ketone).

The term "aliphatic carboxylic acid" refers to a fatty acid, that is, a monocarboxylic acid having a long aliphatic chain. The aliphatic chain may be linear or branched, saturated or unsaturated. The total number of carbon atoms in the carboxylic fatty acid may vary from 4 to 28. Preferred fatty acids are those containing a number of carbon atoms from 8 to 22 carbon atoms, preferably from 12 to 18 carbon atoms. The most preferred fatty acids are caprylic acid (C₈), capric acid (C₁₀), lauric acid (C₁₂), myristic acid (C₁₄), palmitic acid (C₁₆), oleic and stearic acids (C₁₈).

The term "aryl carboxylic acid" refers to a monocarboxylic acid comprising a substituent derived from an aromatic ring. The term aromatic ring encompasses also heteroaromatic rings such as furan, pyridine, thiophene etc. Preferred aryl carboxylic acids are selected from the group consisting of benzene monocarboxylic acid (benzoic acid), toluene monocarboxylic acid, xylene monocarboxylic acid naphthalene monocarboxylic acid and furoic acid. The aromatic ring may contain one or more halogen substituents. The most preferred aryl carboxylic acids are benzoic acid and toluic acid.

A "bivalent metal" refers to a metal capable of forming two chemical bonds with X type ligands (one electron donating neutral ligand). In the same way "trivalent metal" or "tetravalent metal" are metals capable of forming three or four chemical bonds respectively with X type ligands. The preferred metals used in the present invention may be selected from the group consisting of Mg, Ca, Ti, Zr, V, Cr, Mo, W, Mn, Fe, Co, Ni, Cu, Zn, Cd, al, Ga, In, Tl, Ge, Sn, Pb.. More preferably, the metal is Fe or Mg.

The process of the present invention comprises two main steps which are detailed hereafter.

The **first step** comprises a first phase during which the aryl carboxylic acid, the aliphatic carboxylic acid and the metal-containing compound are reacted to yield a mixture of metal carboxylate complexes (also named metal complexes in the present description). It is essential that no free acids be present after this first step in the reaction mixture. For this reason, the amount of metal-containing compound introduced into the acid mixture is calculated by taking into account the valence state of the metal. For a bivalent metal, the amount of metal introduced must be at least 90 % of half of the sum of the number of moles of the aromatic carboxylic acid and the aliphatic carboxylic acid. Let us assume that :
x is the number of moles of the aromatic carboxylic acid;
y is the number of moles of the aliphatic carboxylic acid;
then the number of moles of a bivalent metal should be at least equal to 0.9 [(x+y)/2].

In one preferred embodiment, the number of moles of a bivalent metal introduced into the acid mixture is at least equal to (x+y)/2, preferably it is equal to (x+y)/2. For a trivalent metal, the amount of metal introduced must be at least 90 % of one third of the sum of the number of moles of the aromatic carboxylic acid and the aliphatic carboxylic acid, that is 0.9[(x+y)/3]. For a tetravalent metal, the amount of metal introduced will be at least 90 % of one fourth of the sum of the aromatic carboxylic acid and the aliphatic carboxylic acid, that is 0.9[(x+y)/4]. More generally, for metal with a valence state of z, the amount will be at least 0.9[(x+y)/z].

In addition, mixture of metals with different valence states can be used. This is the case for example for magnetite Fe₃O₄, where there is a mixture of Fe(II) and Fe(III).

In the case of a bivalent metal, the mixture of the aromatic carboxylic acid and the aliphatic carboxylic acid reacts with the bivalent metal to form mainly the following metal carboxylates complexes (I, II and III) :

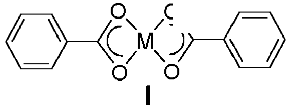

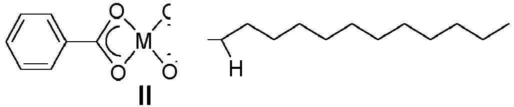

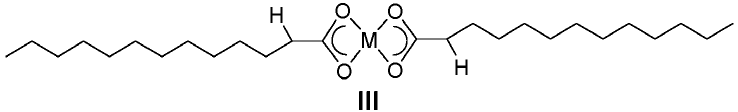

It is essential that all the acids form a complex with the metal.

The first phase of the first step is carried out at a temperature T₁ high enough to convert all the acid to carboxylate complexes. The progressive formation of these complexes may be monitored through Fourier Transform InfraRed spectroscopy (FT-IR) so that the skilled person can adjust temperature T₁ depending on the desired rate of formation of these three complexes. Generally, T₁ ranges from 100°C to 290°C, preferably from 250 to 275°C.

The first step comprises a second phase in which the temperature is increased at a temperature T₂ sufficient to decompose the carboxylate complexes containing at least one aliphatic carboxylate ligand to ketones. In the case of a bivalent metal, the complexes (II) and (III) undergo degradation to ketones : decomposition of the dialiphatic complex (III) leads to the formation of the dialiphatic ketone. Decomposition of the mixed complex (II) leads to the formation of the desired aryl aliphatic ketone. The decomposition of both complexes (II) and (III) releases metal oxide and CO₂. It has been observed that complex (I) is stable and does not decompose into a ketone. The decomposition of both complexes (II) and (III) into the aryl aliphatic ketone and the dialiphatic ketone respectively may be schematized as follows :

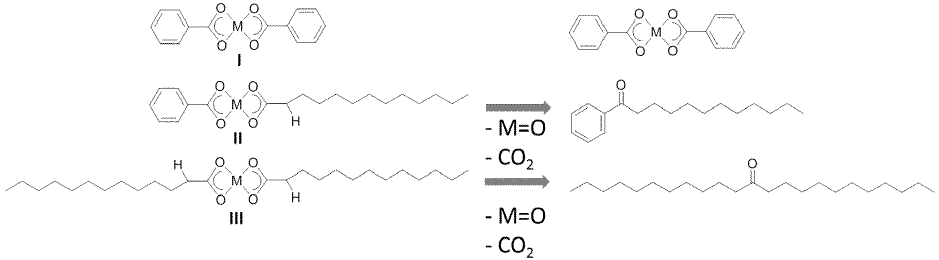

The progressive formation of the ketones may be monitored through Fourier Transform InfraRed spectroscopy (FT-IR) so that the skilled person can adjust temperature T₂ depending on the desired rate of formation of the ketones. Generally, T₂ ranges from 300 to 400°C, preferably from 315 to 400°C.

The time required for complex decomposition generally ranges from 1 to 3 hours, typically less than or equal to 1h30 min.

The **second step** of the process consists in one or several repeated cycles consisting of subsequent additions of the aryl carboxylic acid and the aliphatic carboxylic acid followed by an appropriate reaction time. The aryl carboxylic acid is preferably added before the aliphatic carboxylic acid. It is believed that the metal oxide released by the decomposition of complexes (II) and (III) acts as a catalyst during the subsequent addition(s) of the aryl carboxylic acid and the aliphatic carboxylic acid. Therefore, no new addition of metal-containing compounds is further necessary in the process.

As in the first step, it is important that no free acids be present in the reaction mixture during the subsequent additions of acids. Consequently, appropriate amounts of aryl carboxylic acid and aliphatic carboxylic acid which are to be added in the second step at each cycle are determined such as to compensate the amount of acids that have been consumed through the formation of ketones from decomposition of intermediately formed complexes (II) and (III) at the end of step 1 or at the end of the previous cycle in step 2. For example, in the case of a bivalent metal, the consumption of acids is caused by the decomposition of complexes (II) and (III). Aliphatic carboxylic acid is added in an amount at each cycle which corresponds substantially to the amount of aliphatic carboxylic acid consumed during the formation of the dialiphatic ketone (2 equivalents of aliphatic carboxylic acid are consumed to form 1 equivalent of dialiphatic ketone) and the aryl aliphatic ketone (1 equivalent of aliphatic carboxylic acid is consumed to form 1 equivalent of aryl aliphatic ketone) in the first step or at the end of the previous cycle in the second step. Therefore in theory, all the aliphatic acid added in the first phase of step 1 or at the beginning of the previous cycle in step 2 has been consumed to ketones. Aryl carboxylic acid is added in an amount which corresponds substantially to the amount of aryl carboxylic acid consumed during the formation of the aryl aliphatic ketone (1 equivalent of aryl carboxylic acid is consumed to form 1 equivalent of aryl aliphatic ketone) in the first step or at the end of the previous cycle. By the term "substantially" it is meant that the amount of each acid added at each cycle can deviate from the number of acid consumed during the first step or the previous cycle by plus or minus 30 %, preferably plus or minus 20 %, preferably plus or minus 10 %, preferably plus or minus 5 %, preferably plus or minus 2 %.

The Applicant has calculated for various stoichiometries of aliphatic carboxylic acid, aryl carboxylic acid and bivalent metal involved in the preparation of the reaction mixture of the first step, the theoretical number of equivalents of complexes (I), (II) and (III) formed at the end of the first phase of the first stage as well as the mol% of aryl aliphatic complex (II) relative to the sum of complexes (II) and (III). The results of this determination are reproduced in Table 1 below.

**Table 1 : Number of equivalents of the complexes formed for various ratios of reactants involved in the first step of the process**

| Number of equivalents involved in the first step of the process | | | | Number of equivalents of the complexes formed at stage 1 | | | |
|---|---|---|---|---|---|---|---|
| Condition | x* Aromatic carboxylic acid | y** Aliphatic carboxylic acid | (x+y)/2 (metal) | diaromatic complex (I) | aryl aliphatic complex (II) | dialiphatic complex (III) | mol % of aryl aliphatic complex *** |
| 1 | 0.5 | 1 | 0.75 | 0.08 | 0.33 | 0.33 | **50** |
| 2 | 1 | 1 | 1 | 0.25 | 0.50 | 0.25 | **67** |
| 3 | 1.5 | 1 | 1.25 | 0.45 | 0.60 | 0.20 | **75** |
| 4 | 2 | 1 | 1.5 | 0.67 | 0.67 | 0.17 | **80** |
| 5 | 2.5 | 1 | 1.75 | 0.89 | 0.71 | 0.14 | **83** |
| 6 | 3 | 1 | 2 | 1.13 | 0.75 | 0.13 | **86** |
| 7 | 3.5 | 1 | 2.25 | 1.36 | 0.78 | 0.11 | **88** |
| 8 | 4 | 1 | 2.5 | 1.60 | 0.80 | 0.10 | **89** |
| 9 | 4.5 | 1 | 2.75 | 1.84 | 0.82 | 0.09 | **90** |
| 10 | 5 | 1 | 3 | 2.08 | 0.83 | 0.09 | **91** |

| | | | | | | | |
|---|---|---|---|---|---|---|---|
| * number of equivalents of aryl carboxylic acid ** number of equivalents of aliphatic carboxylic acid *** molar fraction of aryl aliphatic complex (II) relative to the sum of aryl aliphatic complex (II) and dialiphatic complex (III). | | | | | | | |

Assuming that the decomposition rate of complex (II) into the aryl aliphatic ketone is about the same as the decomposition rate of complex (III) into the dialiphatic ketone, one can estimate from the knowledge of the number of moles of complexes (II) and (III) both the number of moles of dialiphatic ketone and the number of moles of aryl aliphatic ketone formed in theory by the ketonization reaction conducted at the end of the second phase in the first step. We can assume that the number of moles of aryl aliphatic ketone will be equal to the number of moles of complex (II) and the number of moles of dialiphatic ketone will be equal to the number of moles of complex (III). Then the amounts of aliphatic and aryl carboxylic acids that need to be added after the first step can be deduced as explained above. As the amounts of carboxylic acids added at each cycle in step 2 is calculated to compensate the amounts of carboxylic acids consumed during the ketonization in the first step or in the previous cycle, it is assumed that at each cycle the ratio of intermediately formed complexes are the same and therefore the amounts of formed ketones are the same. Therefore, the amounts of carboxylic acids added at each cycle in the second step correspond to the amounts of carboxylic acids formed for the formation of aryl aliphatic and dialiphatic ketones during the second phase of step 1) and is the same during the whole process. It can be entirely deduced from the table 1 according to the stoichiometry of aryl carboxylic acid and aliphatic carboxylic acid used in the first step. A detailed example of calculation using the data of Table 1 is provided hereafter in relation with Example 1.1.

The fraction of the metal which is part of the complexes (II) and (III) is released during their decomposition into ketones during the second phase of step 1 or at each cycle of step 2. This metal fraction which can be in the form of metal oxide reacts with each subsequently added aryl carboxylic acid and aliphatic carboxylic acid to regenerate the complex (II) and (III) and water as a by-product.

During each cycle, after subsequent addition of aryl carboxylic acid and aliphatic carboxylic acid and formation of the complexes, the reaction mixture is kept at a temperature sufficient to lead again to the formation of the ketones mixture and metal oxide. The temperature at which the reaction mixture is maintained can be the same as the temperature T₂ of the second phase of first step. It can also be different. Generally, it is convenient to keep the reaction mixture at a temperature substantially equal to T₂, namely T₂ plus or minus 10°C.

Generally, the reaction is kept at a temperature of at least 300°C for a duration ranging from 1 to 3 hours, typically less than or equal to 1h 15 min.

The number of cycles in the second step is not particularly limited. It is however convenient to perform in the second step from three to five cycles. A higher number of repetition may also be envisaged.

Generally, the cycles in the second step of the process are repeated until the ratio of the number of moles of metal over the overall number of moles of acids introduced in the reaction mixture from the beginning of the process is equal to or below 0.25. As explained above, the metal-containing compound is introduced once in the reaction mixture at the beginning of the process while the two acids are progressively introduced in the reaction mixture during the course of the process. Thus, during the reaction, the ratio of the number of moles of metal over the overall number of moles of acids introduced in the reaction mixture from the beginning of the process tends to decrease.

The first and the second step are typically carried out in an inert atmosphere, for example by circulating a stream of N₂ or Ar above the reaction mixture.

Water and carbon dioxide are by-products which are continuously removed from the reaction mixture over the course of the process.

At the end of the process, the desired ketone products are separated from the metal oxide and the metal complex (I) thanks to filtration and/or aqueous washing and/or solvent extraction and/or distillation.

The process of the invention allows preparing an aryl aliphatic ketone with a yield of at least 70 %, preferably at least 80 %, preferably at least 95 % and up to 99 %.

The process of the invention allows preparing an aryl aliphatic ketone with a selectivity of at least 55 %, preferably at least 80 %, preferably at least 85 %, preferably at least 95 %.

In one preferred embodiment, the process of the invention allows preparing an aryl aliphatic ketone with a selectivity of at least 55 % for an overall molar ratio of aryl carboxylic acid over aliphatic carboxylic acid not higher than 0.37.

In one preferred embodiment, the process of the invention allows preparing an aryl aliphatic ketone with a selectivity of at least 85 % for a molar ratio of aryl carboxylic acid over aliphatic carboxylic acid not higher than 0.83.

In one preferred embodiment, the process of the invention allows preparing an aryl aliphatic ketone with a selectivity of at least 99 % for a molar ratio of aryl carboxylic acid over aliphatic carboxylic acid not higher than 1.75.

This high selectivity is desirable to reduce manufacturing costs and to decrease complexity of downstream processes.

In addition to obtaining a high selectivity and a high yield, the process of the invention provides the following advantages :
- it does not require any additional solvent.
- the catalyst does not need to be used in the form of nanoparticles.
- it can be performed in a relatively short reaction times (less than or equal to 10 hours, generally less than or equal to 8 hours, less than or equal to 6 hours).
- it can be conducted at atmospheric pressure.

The aryl aliphatic ketones prepared by the process of the invention are seen as valuable intermediates to be transformed into new surfactants. They are potential candidates for the replacement of widely used Nonylphenol ethoxylates (NPE). Indeed, nowadays, nonylphenol ethoxylates raise concerns because of their reprotoxicity and also because they are considered as endocrine disruptors.

The aryl aliphatic ketones prepared through the process of the present invention may be used as reactants in a single or a multiple chemical reaction scheme. The end product may act as an emulsifier, a textile auxiliary, a dyeing additive, a detergent, a dispersant, a softener, a crude oil emulsifier. It may be used in sectors of industry such as detergents, personal care products, textiles, metallurgical industry, paper making, petroleum, pesticides, pharmacy, printing, synthetic rubbers, aqueous emulsions, coatings, inks, glue and plastics.

### EXAMPLES

All the reactions were conducted under a strictly oxygen-free atmosphere (under argon).

### 1-Preparation of phenylhexadecan-1-one :

### 1.1 Using 0.37 equivalent of benzoic acid and 1 equivalent of palmitic acid :

In a round bottom flask equipped with a Dean-Stark apparatus, an insulated addition funnel filled with 50 g of melted palmitic acid (195.1 mmol), a second solid addition funnel filled with 8.6 g of benzoic acid (70.5 mmol), a temperature probe and a mechanical stirrer are added 2 g of metal iron (35.8 mmol).

### - Initial complex generation and decomposition :

Then, 12.5 g (48.8 mmol) of palmitic acid followed by 2.9 g (23.8 mmol) of benzoic acid are added into the reaction medium and the mixture is allowed to stir at 250°C during 2h. As it can be easily calculated, the amount of iron used in this first step corresponds nearly to half of the sum of the number of moles of palmitic acid and the number of moles of benzoic acid.

During this first step, Fe⁽⁰⁾ is converted to a mixture of Fe^{(II)} carboxylate complexes with concomitant release of hydrogen gas. After complete conversion of acids toward complexes (that can be followed thanks to FTIR analysis), the reaction media is heated to 315°C and allowed to stir at this temperature during 1h30 in order to complete the transformation of complexes II and III toward the corresponding ketones. Release of CO₂ gas is observed at this stage.

### - Cycles of acid addition and ketonization :

Thereafter, 1.9 g of benzoic acid (15.6 mmol) and 12.5 g of palmitic acid (48.8 mmol) are added into the reactor and the mixture is allowed to stir at 315°C. The reaction progress can be followed by FTIR analysis which showed that conversion of the complexes toward ketones was achieved after 1h15 of reaction time after the end of the addition.

Two additional cycles each consisting of acids addition (12.5 g of palmitic acid and 1.9 g of benzoic acid) followed by heating at 315°C during 1h15min were performed.

Finally, the reaction progress was monitored thanks to FTIR in order to ensure that complete conversion of complexes II and III was reached. This requires usually an additional 0h30 of stirring after the last cycle.

### - Work-up :

After reaction completion, the reaction mixture is cooled down at 40°C and chloroform (CHCl₃) is added into the mixture to dissolve products. In order to separate the desired product from iron oxide and the remaining iron carboxylate complex, the chloroform mixture is filtered over a silica plug and the product eluted with around 1.5 L of CHCl₃. After evaporation of the solvent, 47.71 g of product (91 % yield relative to fatty acids) is obtained as a light brown powder constituted of 55 mol % of phenylhexadecan-1-one and 45 mol % of hentriacontan-16-one (palmitone).

One can easily determine in theory by calculation the number of moles of palmitic acid and the number of moles of benzoic acid that have been consumed and converted into ketones during each decomposition phase of complexes (II) and (III). The calculation will be provided hereunder. A similar calculation may be made for all the other examples.

The ratio of the number of moles of aryl carboxylic acid (benzoic acid) to the number of aliphatic carboxylic acid (palmitic acid) introduced during the first step of complex generation is 23.8 mmol / 48.8 mmol ∼ 0.5 which means that 0.5 equivalent of benzoic acid is used for 1 equivalent of palmitic acid.

Table 1 above indicates at row entitled "condition 1" that for a ratio aryl carboxylic acid/aliphatic carboxylic acid of 0.5/1, the following equivalents are formed :
- 0.08 equivalent of the diaromatic complex (complex I) is formed;
- 0.33 equivalent of the aryl aliphatic complex (complex II) is formed;
- 0.33 equivalent of the dialky complex (complex III) is formed.

We can then calculate the number of moles of each complex formed knowing that 1 equivalent represents in this case 48.8 mmol.
- 0.08 × 48.8 = 3.90 mmol of the diaromatic complex (complex I) are formed;
- 0.33 × 48.8 = 16.27 mmol of the aryl aliphatic complex (complex II) are formed;
- 0.33 × 48.8 = 16.27 mmol equivalent of the dialiphatic complex (complex III) is formed.

The formation of 1 mole of the aryl aliphatic complex (complex II) requires 1 mole of aryl carboxylic acid and 1 mole of aliphatic carboxylic acid.

The formation of 1 mole of the dialiphatic complex (complex III) requires 2 moles of aliphatic carboxylic acid.

Complex I does not decompose into a ketone.

One can thus determine that the formation 16.27 mmol of the aryl aliphatic ketone has required 16.27 mmol of aryl carboxylic acid and 16.27 mmol of aliphatic carboxylic acid. One can also determine that the formation 16.27 mmol of the dialiphatic ketone has required 2 × 16.27 = 32.54 mmol of aliphatic carboxylic acid.

One finds that the number of moles of aryl carboxylic acid that was converted into the aryl aliphatic ketone was 16.27 mmol.

One finds that the number of moles of aliphatic carboxylic acid that was consumed to form the aryl aliphatic ketone and the dialiphatic ketone was 32.54 + 16.27 = 48.8 mmol meaning the entire amount of aliphatic carboxylic acid introduced during the step of complex generation and decomposition.

The experimental amounts of 15.6 and 48.8 mmol used during each cycle of step 2 in the above example are consistent with these two theoretical values of 16.27 and 48.8 mmol.

### 1.2 Using 0.83 equivalent of benzoic acid and 1 equivalent of palmitic acid :

The reaction is carried out following the protocol described in Example 1.1 with a total amount of 50 g of palmitic acid (195.1 mmol), 19.8 g of benzoic acid (162.2 mmol) and 3.4 g of Fe⁽⁰⁾ (60.8 mmol).

During initial complex generation and decomposition, 3.4 g (60.8 mmol) of Fe is reacted with 12.5 g (48.8 mmol) of palmitic acid and 9.0 g (73.7 mmol) of benzoic acid.

During the 3 cycles of acids addition and decomposition, 12.5 g (48.8 mmol) of palmitic acid and 3.6 g (29.5 mmol) of benzoic acid are added at each cycle.

The product is obtained as a brown powder (51.84 g, 91 % isolated yield relative to the fatty acid) and consists of 85 mol % of aryl aliphatic ketone and 15 mol % of hentriacontan-16-one.

### 1.3 Using 1.75 equivalent of benzoic acid and 1 equivalent of palmitic acid :

The reaction is carried out following the protocol described in Example 1.1 with a total amount of 32.8 g of palmitic acid (128.0 mmol), 27.2 g of benzoic acid (222.9 mmol) and 4.9 g of Fe (87.6 mmol).

During initial complex generation and decomposition, 4.9 g (87.6 mmol) of Fe is reacted with 8.2 g (32.0 mmol) of palmitic acid and 17.6 g (144.2 mmol) of benzoic acid.

During the 3 cycles of acids addition and decomposition, 8.2 g (32.0 mmol) of palmitic acid and 3.2 g (26.2 mmol) of benzoic acid are added at each cycle.

The product is obtained as a brown powder (33.75 g, 83 % isolated yield relative to the fatty acid) and consists of 99 mol % of cross adduct and only 1 mol % of hentriacontan-16-one.

### 2-Preparation of 1-phenyldodecan-1-one :

### 2.1 Using 0.83 equivalent of benzoic acid and 1 equivalent of lauric acid :

The reaction is carried out following the protocol described in Example 1.1 with a total amount of 39.2 g of lauric acid (195.8 mmol), 19.8 g of benzoic acid (162.2 mmol) and 3.4 g of Fe⁽⁰⁾ (60.8 mmol).

During initial complex generation and decomposition, 3.4 g (60.8 mmol) of Fe is reacted with 9.8 g (48.9 mmol) of lauric acid and 9.0 g (73.7 mmol) of benzoic acid.

During the 3 cycles of acids addition and decomposition, 9.8 g (48.9 mmol) of lauric acid and 3.6 g (29.5 mmol) of benzoic acid are added at each cycle.

The product is obtained as a brown powder (40.3g, 87 % isolated yield relative to the fatty acid) and consists of 87 mol % of cross adduct and 13 mol % of tricosan-12-one.

### 2.2 Using 1.75 equivalent of benzoic acid and 1 equivalent of lauric acid :

The reaction is carried out following the protocol described in Example 1.1 with a total amount of 25.6 g of lauric acid (127.9 mmol), 27.2 g of benzoic acid (222.9 mmol) and 4.9 g of Fe⁽⁰⁾ (87.6 mmol).

During initial complex generation and decomposition, 4.9 g (87.6 mmol) of Fe is reacted with 6.4 g (31.9 mmol) of lauric acid and 17.6 g (144.2 mmol) of benzoic acid.

During the 3 cycles of acids addition and decomposition, 6.4 g (31.9 mmol) of lauric acid and 3.2 g (26.2 mmol) of benzoic acid are added at each cycle.

The product is obtained as a brown powder (23.84g, 71 % isolated yield relative to the fatty acid) and consists of 99 mol % of cross adduct and 1 mol % of tricosan-12-one.

### 3-Preparation of 1-(furan-2-yl)dodecan-1-one :

### 3.1 Using 0.83 equivalent of 2-furoic acid and 1 equivalent of lauric acid :

The reaction is carried out following the protocol described in Example 1.1 with a total amount of 39.2 g of lauric acid (195.8 mmol), 18.1 g of 2-furoic acid (161.6 mmol) and 3.4 g of Fe (60.8 mmol).

During initial complex generation and decomposition, 3.4 g (60.8 mmol) of Fe⁽⁰⁾ is reacted with 9.8 g (48.9 mmol) of lauric acid and 8.2 g (73.2 mmol) of 2-furoic acid.

During the 3 cycles of acids addition and decomposition, 9.8 g (48.9 mmol) of lauric acid and 3.3 g (29.5 mmol) of 2-furoic acid are added at each cycle.

The product is obtained as a black viscous oil (23.84g, 80 % isolated yield relative to the fatty acid) and consists of 24 mol % of cross adduct and 76 mol % of tricosan-12-one.

### 3.2 Using 1.75 equivalent of 2-furoic acid and 1 equivalent of lauric acid :

The reaction is carried out following the protocol described in Example 1.1 with a total amount of 25.6 g of lauric acid (127.9 mmol), 25.5 g of 2-furoic acid (228.0 mmol) and 4.91 g of Fe (87.8 mmol).

During initial complex generation and decomposition, 4.91 g (87.8 mmol) of Fe is reacted with 6.4 g (31.9 mmol) of lauric acid and 16.1 g (144.0 mmol) of 2-furoic acid.

During the 3 cycles of acids addition and decomposition, 6.4 g (31.9 mmol) of lauric acid and 3.1 g (27.7 mmol) of 2-furoic acid are added at each cycle.

The product is obtained as a black viscous oil (22.96g, 91 % isolated yield relative to the fatty acid) and consists of 49 mol % of cross adduct and 51 mol % of tricosan-12-one.

### 4-Preparation of 1-(furan-3-yl)dodecan-1-one :

### 4.1 Using 0.83 equivalent of 3-furoic acid and 1 equivalent of lauric acid :

The reaction is carried out following the protocol described in Example 1.1 with a total amount of 39.2 g of lauric acid (195.8 mmol), 18.1 g of 3-furoic acid (161.9 mmol) and 3.4 g of Fe (60.8 mmol).

During initial complex generation and decomposition, 3.4 g (60.8 mmol) of Fe is reacted with 9.8 g (48.9 mmol) of lauric acid and 8.2 g (73.2 mmol) of furoic acid.

During the 3 cycles of acids addition and decomposition, 9.8 g (48.9 mmol) of lauric acid and 3.3 g (29.5 mmol) of furoic acid are added at each cycle.

The product is obtained as a black viscous oil (36.5g, 91 % isolated yield relative to the fatty acid) and consists of 58 mol % of cross adduct and 42 mol % of tricosan-12-one.

The results in terms of isolated yields and selectivity obtained for the above examples are summarized in the following table :

**Table 2 : Values of yields and selectivities**

| Example | Aromatic acid | Fatty acid | % mol Fe (% wt.) | Molar ratio aromatic acid: fatty acid | Yield % in ketone | Selectivity (mol % of aryl aliphatic ketone) |
|---|---|---|---|---|---|---|
| 1.1 | benzoic acid | palmitic acid | 13 (3.4) | 0.37:1 | 91 | 55 |
| 1.2 | benzoic acid | palmitic acid | 17 (4.9) | 0.83:1 | 91 | 85 |
| 1.3 | benzoic acid | palmitic acid | 25 (8.2) | 1.75 : 1 | 83 | 99 |
| 2.1 | benzoic acid | lauric acid | 17 (5.8) | 0.83 : 1 | 87 | 87 |
| 2.2 | benzoic acid | lauric acid | 25 (9.3) | 1.75 : 1 | 71 | 99 |
| 3.1 | 2-furoic acid | lauric acid | 17 (5.9) | 0.83 : 1 | 80 | 24 |
| 3.2 | 2-furoic acid | lauric acid | 24(9.3) | 1.75 : 1 | 91 | 49 |
| 4.1 | 3-furoic acid | lauric acid | 17 (5.9) | 0.83 : 1 | 91 | 58 |

### 5- Control experiment :

### Stoichiometric reaction between Fe-(laurate)₂ complex and Fe-(benzoate)₂ complex :

### - Preparation of Fe-laurate complex :

In a 50 mL round bottom flask equipped with a magnetic stirrer, under an inert argon atmosphere, 1.8 g of Fe (32.2 mmol) is reacted at 240°C with 12.5 g of lauric acid (62.4 mmol). The reaction is monitored thanks to FTIR analysis. When complete conversion of fatty acid toward the desired complex is observed, the reaction mixture is cooled down at room temperature and the product is washed several times with acetone in order to remove traces of remaining acid, ketone and by-products.
8.62 g of complex has been obtained as a brown powder (61 % yield).

### - Preparation of Fe-benzoate complex :

In a 50 mL round bottom flask equipped with a magnetic stirrer, under an inert argon atmosphere, 1.8 g of Fe (32.2 mmol) is reacted at 250°C with 7.6 g of benzoic acid (62.3 mmol). The reaction is monitored thanks to FTIR analysis. When complete conversion of benzoic acid toward the desired complex is observed the reaction mixture is cooled down at room temperature and the product is washed several times with acetone in order to remove traces of remaining acid and by-products.
4.33 g of complex has been obtained as a brown powder (47 % yield).

### - Stoichiometric reaction between the aromatic complex and the aliphatic complex :

In a 50 mL round bottom flask equipped with a mechanical stirrer, under an inert argon atmosphere, 4.3 g of Fe-benzoate complex (62.3 mmol) is reacted at 315°C with 6.6 g of Fe-laurate complex (62.3 mmol). The reaction is monitored thanks to FTIR analysis.

At the end of the reaction, when complete conversion of complex toward ketones has been observed, the reaction mixture is cooled down at 40°C and CHCl₃ is added into the mixture to dissolve products.

In order to separate the desired product from iron oxide and remaining iron carboxylate complex, the chloroform mixture is filtered over a silica plug and the product eluted with around 1.5 L of CHCl₃. After evaporation of the solvent, 5.41 g of product (77 % yield) is obtained as a light brown powder constituted of 86 mol % of 1-phenyldodecan-1-one and 14 mol % of tricosan-12-one.

This control experiment shows that :
1) Carboxylate ligands exchange around Fe center is rapid in the conditions of the invention and faster than the ketonization step that converts Fe complexes toward the ketones.
2) Selectivity in this 1:1 stoichiometric reaction between lauric acid and benzoic acid is 86 %. In comparison, a similar selectivity has been obtained in the present invention using only 0.83 equivalent of benzoic acid versus aliphatic acid showing that under this implementation, selectivity toward the aryl aliphatic ketone is improved using catalytic quantities of Fe.

## Claims

1. A process for the catalytic decarboxylative cross-ketonisation of aryl- and aliphatic carboxylic acids comprising the steps of:
a) providing a mixture containing :
i) an aryl carboxylic acid;
ii) an aliphatic carboxylic acid;
iii) a metal-containing compound;
in which the number of moles of the metal in the mixture is at least equal to 90 % of the sum of the number of moles of aryl carboxylic acid and the number of moles of aliphatic carboxylic acid divided by the valency of the metal
said mixture being substantially free of any added solvent;
b) heating the mixture at a temperature sufficient to form metal carboxylates;
c) further heating the mixture at a temperature sufficient to form a dialiphatic ketone and an aryl aliphatic ketone;
d) adding to the reaction mixture of step c) :
i) aryl carboxylic acid in an amount which corresponds substantially to the amount of aryl carboxylic acid consumed during the formation of the aryl aliphatic ketone in step c);
ii) aliphatic carboxylic acid in an amount which corresponds substantially to the amount of aliphatic carboxylic acid consumed during the formation of the aryl aliphatic ketone and the dialiphatic ketone in step c);
maintaining the mixture at a temperature sufficient to continue forming the dialiphatic ketone and the aryl aliphatic ketone;
e) optionally repeating step d).

2. The process of claim 1, wherein :
x is the number of moles of aryl carboxylic acid
y is the number of moles of aliphatic carboxylic acid, and
the number of moles of a metal of valence z in the mixture of step a) is within the range of from 0.9[(x+y)/z] to 1.1[(x+y)/z], preferably from 0.95[(x+y)/z] to 1.05[(x+y)/z], more preferably from 0.98[(x+y)/z] to 1.02[(x+y)/z], more preferably from 0.99[(x+y)/z] to 1.01[(x+y)/z].

3. The process according to claim 1, wherein the metal is iron.

4. The process of claim 3, wherein the iron-containing compound is selected from the group consisting of iron metal, bivalent Fe^{(II)} compounds, trivalent Fe^{(III)} compounds, compounds in which iron is present in both bivalent Fe^{(II)} and bivalent Fe^{(III)} oxidation states, such as magnetite Fe₃O₄.

5. The process of any of the preceding claims, wherein step b) is carried out at a temperature T₁ from 225°C to 290°C, preferably from 250°C to 275°C.

6. The process of any of the preceding claims, wherein step c) and/or step d) is carried out at a temperature T₂ from 300°C to 400°C, preferably from 315°C to 400°C.

7. The process of any of the preceding claims, wherein the molar ratio of the total amount of aryl carboxylic acid and the aliphatic carboxylic acid ranges from 0.3 to 1.8.

8. The process of any of the preceding claims, wherein heating in step b) is carried out for a duration ranging from 1 to 6 h, preferably for a duration less than or equal to 3h.

9. The process of any of the preceding claims, wherein heating in step c) is carried out for a duration ranging from 1 to 4 h, preferably for a duration less than or equal to 1,5h.

10. The process of any of the preceding claims, wherein heating in step d) is carried out for a duration ranging from 1 to 4 h, preferably for a duration less than or equal to 1,25 h.

11. The process of any of the preceding claims, being carried out for a duration less than or equal to 20 hours, preferably for a duration less than or equal to 8 hours.

12. The process of any of the preceding claims, wherein the aryl carboxylic acid is selected from the group consisting of benzoic acid, furoic acid, o-toluic acid, m-toluic acid and p-toluic acid.

13. The process of any of the preceding claims, wherein the aliphatic carboxylic acid is a fatty acid.

14. The process according to claim 13, wherein the fatty acid contains from 8 to 18 carbon atoms.

15. A process for the preparation of at least one end compound from at least one aryl aliphatic ketone, said method comprising :
- synthesizing the aryl aliphatic ketone through the process of any of the preceding claims,
- causing the aryl aliphatic ketone to react in accordance with a single or multiple chemical reaction scheme involving at least one reagent other than the aryl aliphatic ketone, wherein at least one product of the chemical reaction scheme is the end compound that is not further caused to be chemically converted into another compound.

## Patentansprüche

1. Verfahren zur katalytischen decarboxylierenden Kreuzketonisierung von Aryl- und aliphatischen Carbonsäuren, umfassend die Schritte:
a) Bereitstellen eines Gemischs, das enthält:
i) eine Arylcarbonsäure;
ii) eine aliphatische Carbonsäure;
iii) eine metallhaltige Verbindung;
wobei die Molzahl des Metalls in dem Gemisch wenigstens gleich 90 % der Summe der Molzahl an Arylcarbonsäure und der Molzahl an aliphatischer Carbonsäure, geteilt durch die Wertigkeit des Metalls, ist,
wobei das Gemisch im Wesentlichen frei von zugegebenem Lösungsmittel ist;
b) Erhitzen des Gemischs auf eine Temperatur, die ausreicht, um Metallcarboxylate zu bilden;
c) weiteres Erhitzen des Gemischs auf eine Temperatur, die ausreicht, um ein dialiphatisches Keton und ein arylaliphatisches Keton zu bilden;
d) zu dem Reaktionsgemisch von Schritt c) Zugeben von:
i) Arylcarbonsäure in einer Menge, die im Wesentlichen der Menge an Arylcarbonsäure entspricht, die bei der Entstehung des arylaliphatischen Ketons bei Schritt c) verbraucht wird;
ii) aliphatische Carbonsäure in einer Menge, die im Wesentlichen der Menge an aliphatischer Carbonsäure entspricht, die bei der Entstehung des arylaliphatischen Ketons und des dialiphatischen Ketons bei Schritt c) verbraucht wird;
Halten des Gemischs bei einer Temperatur, die ausreicht, um die Entstehung des dialiphatischen Ketons und des arylaliphatischen Ketons fortzusetzen;
e) gegebenenfalls Wiederholen von Schritt d).

2. Verfahren gemäß Anspruch 1, wobei:
x die Molzahl an Arylcarbonsäure ist,
y die Molzahl an aliphatischer Carbonsäure ist und
die Molzahl eines Metalls mit der Wertigkeit z in dem Gemisch von Schritt a) in dem Bereich von 0,9[(x + y)/z] bis 1,1[(x + y)/z], vorzugsweise von 0,95[(x + y)/z] bis 1,05[(x + y)/z], bevorzugter von 0,98[(x + y)/z] bis 1,02[(x + y)/z], bevorzugter von 0,99[(x + y)/z] bis 1,01[(x + y)/z], liegt.

3. Verfahren gemäß Anspruch 1, wobei das Metall Eisen ist.

4. Verfahren gemäß Anspruch 3, wobei die eisenhaltige Verbindung ausgewählt ist aus der Gruppe bestehend aus Eisenmetall, zweiwertigen Fe^{(II)}-Verbindungen, dreiwertigen Fe^{(III)}-Verbindungen, Verbindungen, in denen Eisen sowohl in zweiwertigen Fe^{(II)}- als auch in zweiwertigen Fe^{(III)}-Oxidationszuständen vorliegt, wie z. B. Magnetit Fe₃O₄.

5. Verfahren gemäß einem der vorstehenden Ansprüche, wobei Schritt b) bei einer Temperatur T₁ von 225 °C bis 290 °C, vorzugsweise von 250 °C bis 275 °C, durchgeführt wird.

6. Verfahren gemäß einem der vorstehenden Ansprüche, wobei Schritt c) und/oder Schritt d) bei einer Temperatur T₂ von 300 °C bis 400 °C, vorzugsweise von 315 °C bis 400 °C, durchgeführt wird.

7. Verfahren gemäß einem der vorstehenden Ansprüche, wobei das Molverhältnis der Gesamtmenge an Arylcarbonsäure und der aliphatischen Carbonsäure in dem Bereich von 0,3 bis 1,8 liegt.

8. Verfahren gemäß einem der vorstehenden Ansprüche, wobei das Erhitzen bei Schritt b) für eine Dauer in dem Bereich von 1 bis 6 h durchgeführt wird, vorzugsweise für eine Dauer von kleiner als oder gleich 3 h.

9. Verfahren gemäß einem der vorstehenden Ansprüche, wobei das Erhitzen bei Schritt c) für eine Dauer in dem Bereich von 1 bis 4 h durchgeführt wird, vorzugsweise für eine Dauer von kleiner als oder gleich 1,5 h.

10. Verfahren gemäß einem der vorstehenden Ansprüche, wobei das Erhitzen bei Schritt d) für eine Dauer in dem Bereich von 1 bis 4 h durchgeführt wird, vorzugsweise für eine Dauer von kleiner als oder gleich 1,25 h.

11. Verfahren gemäß einem der vorstehenden Ansprüche, das für eine Dauer von kleiner als oder gleich 20 Stunden durchgeführt wird, vorzugsweise für eine Dauer von kleiner als oder gleich 8 Stunden.

12. Verfahren gemäß einem der vorstehenden Ansprüche, wobei die Arylcarbonsäure ausgewählt ist aus der Gruppe bestehend aus Benzoesäure, Furoesäure, o-Toluylsäure, m-Toluylsäure und p-Toluylsäure.

13. Verfahren gemäß einem der vorstehenden Ansprüche, wobei die aliphatische Carbonsäure eine Fettsäure ist.

14. Verfahren gemäß Anspruch 13, wobei die Fettsäure von 8 bis 18 Kohlenstoffatomen enthält.

15. Verfahren zur Herstellung wenigstens einer Endverbindung aus wenigstens einem arylaliphatischen Keton, wobei das Verfahren umfasst:
- Synthetisieren des arylaliphatischen Ketons durch das Verfahren gemäß einem der vorstehenden Ansprüche,
- Bewirken der Reaktion des arylaliphatischen Ketons gemäß einem chemischen Einzel- oder Mehrfachreaktionsschema unter Beteiligung wenigstens eines von dem arylaliphatischen Keton verschiedenen Reagens, wobei wenigstens ein Produkt des chemischen Reaktionsschemas die Endverbindung ist, von keine chemische Umwandlung in eine andere Verbindung bewirkt wird.

## Revendications

1. Procédé pour la cétonisation croisée décarboxylative catalytique d'acides arylcarboxyliques et d'acides carboxyliques aliphatiques comprenant les étapes de :
a) mise à disposition d'un mélange contenant :
i) un acide arylcarboxylique ;
ii) un acide carboxylique aliphatique ;
iii) un composé contenant un métal ;
dans lequel le nombre de moles du métal dans le mélange est au moins égal à 90 % de la somme du nombre de moles d'acide arylcarboxylique et du nombre de moles d'acide carboxylique aliphatique divisée par la valence du métal
ledit mélange étant sensiblement exempt de quelconque solvant ajouté ;
b) chauffage du mélange à une température suffisante pour former des carboxylates de métal ;
c) poursuite du chauffage du mélange à une température suffisante pour former une cétone dialiphatique et une arylcétone aliphatique ;
d) ajout au mélange réactionnel de l'étape c) de :
i) un acide arylcarboxylique en une quantité qui correspond sensiblement à la quantité d'acide arylcarboxylique consommée pendant la formation de l'arylcétone aliphatique dans l'étape c) ;
ii) un acide carboxylique aliphatique en une quantité qui correspond sensiblement à la quantité d'acide carboxylique aliphatique consommée pendant la formation de l'arylcétone aliphatique et de la cétone dialiphatique dans l'étape c) ;
maintien du mélange à une température suffisante pour continuer la formation de la cétone dialiphatique et de l'arylcétone aliphatique ; e)éventuellement répétition de l'étape d).

2. Procédé selon la revendication 1,
x étant le nombre de moles d'acide arylcarboxylique
y étant le nombre de moles d'acide carboxylique aliphatique, et
le nombre de moles d'un métal de valence z dans le mélange de l'étape a) se situant dans la plage de 0,9[(x + y)/z] à 1,1[ (x + y)/z], préférablement de 0,95[(x + y)/z] à 1,05[(x + y)/z], plus préférablement de 0,98[(x + y)/z] à 1,02[(x + y)/z], plus préférablement de 0,99[(x + y)/z] à 1,01[(x + y)/z].

3. Procédé selon la revendication 1, le métal étant le fer.

4. Procédé selon la revendication 3, le composé contenant du fer étant choisi dans le groupe constitué par le fer métallique, les composés de Fe^{(II)} divalent, les composés de Fe^{(III)} trivalent, des composés dans lequel le fer est présent à la fois dans des états d'oxydation de Fe^{(II)} divalent et de Fe^{(III)} divalent, tels que la magnétite Fe₃O₄.

5. Procédé selon l'une quelconque des revendications précédentes, l'étape b) étant mise en œuvre à une température T₁ de 225 °C à 290 °C, préférablement de 250 °C à 275 °C.

6. Procédé selon l'une quelconque des revendications précédentes, l'étape c) et/ou l'étape d) étant mise (s) en œuvre à une température T₂ de 300 °C à 400 °C, préférablement de 315 °C à 400 °C.

7. Procédé selon l'une quelconque des revendications précédentes, le rapport molaire de la quantité totale d'acide arylcarboxylique et de l'acide carboxylique aliphatique se situant dans la plage de 0,3 à 1,8.

8. Procédé selon l'une quelconque des revendications précédentes, le chauffage dans l'étape b) étant mis en œuvre pendant une durée se situant dans la plage de 1 à 6 h, préférablement pendant une durée inférieure ou égale à 3 h.

9. Procédé selon l'une quelconque des revendications précédentes, le chauffage dans l'étape c) étant mis en œuvre pendant une durée se situant dans la plage de 1 à 4 h, préférablement pendant une durée inférieure ou égale à 1,5 h.

10. Procédé selon l'une quelconque des revendications précédentes, le chauffage dans l'étape d) étant mis en œuvre pendant une durée se situant dans la plage de 1 à 4 h, préférablement pendant une durée inférieure ou égale à 1,25 h.

11. Procédé selon l'une quelconque des revendications précédentes, mis en œuvre pendant une durée inférieure ou égale à 20 heures, préférablement pendant une durée inférieure ou égale à 8 heures.

12. Procédé selon l'une quelconque des revendications précédentes, l'acide arylcarboxylique étant choisi dans le groupe constitué par l'acide benzoïque, l'acide furoïque, l'acide o-toluique, l'acide m-toluique et l'acide p-toluique.

13. Procédé selon l'une quelconque des revendications précédentes, l'acide carboxylique aliphatique étant un acide gras.

14. Procédé selon la revendication 13, l'acide gras contenant de 8 à 18 atomes de carbone.

15. Procédé pour la préparation d'au moins un composé final à partir d'au moins une arylcétone aliphatique, ledit procédé comprenant :
- la synthèse de l'arylcétone aliphatique par le procédé selon l'une quelconque des revendications précédentes,
- le fait d'amener l'arylcétone aliphatique à réagir conformément à un schéma de réaction unique ou multiple impliquant au moins un réactif différent de l'arylcétone aliphatique, au moins un produit du schéma de réaction chimique étant le composé final qui n'est pas amené à être converti chimiquement en un autre composé.
